# FASCICULE DE BREVET EUROPEEN

(11) **EP 3 365 674 B1**
(45) Date de publication et mention de la délivrance du brevet: **14.08.2019**
(21) Numéro de dépôt: 16785127.8
(22) Date de dépôt: 20.10.2016
(51) Int. Cl.: G01N 29/036, G01N 33/00, G01N 29/02

(54) **UTILISATION D'OLIGOSILSESQUIOXANES OCTAÉDRIQUES FONCTIONNALISÉS COMME MATÉRIAUX SENSIBLES DANS DES CAPTEURS CHIMIQUES DESTINÉS À DÉTECTER LA PRÉSENCE DE STUPÉFIANTS DANS UN MILIEU GAZEUX**
VERWENDUNG VON FUNKTIONALISIERTEN OKTAEDRISCHEN OLIGOSILSESQUIOXANEN ALS SENSITIVE MATERIALIEN IN CHEMISCHEN SENSOREN ZUR DETEKTION DES VORHANDENSEINS VON NARKOSEMITTEL IN EINEM GASMEDIUM
USE OF FUNCTIONALIZED OCTAHEDRAL OLIGOSILSESQUIOXANES AS SENSITIVE MATERIALS IN CHEMICAL SENSORS FOR DETECTING THE PRESENCE OF NARCOTICS IN A GAS MEDIUM

(30) Priorité: 20.10.2015 FR 1559995
(43) Date de publication de la demande: 29.08.2018
(73) Titulaire: Commissariat à l'Energie Atomique et aux Energies Alternatives, 75015 Paris (FR)
(72) Inventeur: HAIRAULT, Lionel, 37150 Blere (FR); GREGIS, Geoffrey, 25000 Besançon (FR); CHAMPEAU, Mathilde, 35200 Rennes (FR); MINOT, Benoît, 37700 Saint-Pierre-des-Corps (FR)
(74) Mandataire: Brevalex
(86) Numéro de dépôt international: PCT/EP2016/075238
(87) Numéro de publication internationale: WO 2017/068046

(56) Documents cités:
- US-A1- 2005 090 015
- MARCEL BENZ ET AL: "High Temperature Mass Detection Using a Carbon Nanotube Bilayer Modified Quartz Crystal Microbalance as a GC Detector", ANALYTICAL CHEMISTRY, vol. 87, no. 5, 3 mars 2015 (2015-03-03), pages 2779-2787, XP055285738, ISSN: 0003-2700, DOI: 10.1021/ac504101a
- ADAM MCCLUSKEY ET AL: "Molecularly imprinted polymers (MIPs): sensing, an explosive new opportunity?", ORGANIC & BIOMOLECULAR CHEMISTRY, vol. 5, no. 20, 1 janvier 2007 (2007-01-01), page 3233, XP055000148, ISSN: 1477-0520, DOI: 10.1039/b708660a
- ROBERT E. SPEIGHT ET AL: "A Survey of the 2010 Quartz Crystal Microbalance Literature", JOURNAL OF MOLECULAR RECOGNITION, vol. 25, no. 9, 1 septembre 2012 (2012-09-01), pages 451-473, XP055043098, ISSN: 0952-3499, DOI: 10.1002/jmr.2209

## Description

### DOMAINE TECHNIQUE

La présente invention se rapporte au domaine de la détection de stupéfiants.

Plus spécifiquement, elle se rapporte à l'utilisation d'oligosilsesquioxanes octaédriques fonctionnalisés comme matériaux sensibles dans des capteurs chimiques qui sont destinés à détecter la présence de stupéfiants, de leurs précurseurs et/ou de leurs produits de dégradation dans un milieu gazeux et, en particulier, d'opiacés, de cocaïne, de cannabinoïdes, d'amphétamines, d'acide lysergique de diéthylamide (ou LSD), de cathine et de cathinone (qui sont deux alcaloïdes présents dans le khat), de dérivés synthétiques de la cathinone, d'acide γ-hydroxybutyrique (aussi appelée « *drogue des violeurs* »), de kétamine, de nitrite d'isoamyle et de nitrite de n-amyle (qui entrent dans la composition des poppers), ainsi que de leurs isomères, leurs sels, leurs esters et de leurs éthers.

L'invention trouve notamment application dans la lutte contre le trafic des stupéfiants, aussi appelé narcotrafic.

Elle trouve également application dans le dépistage de la consommation illicite de stupéfiants dans des lieux à usage collectif, qu'ils soient publics ou privés, comme les établissements scolaires (collèges, lycées, universités, etc), les installations sportives, les transports publics, les gares, les aéroports, les administrations, les entreprises, les boites de nuit, etc, ainsi que dans le cadre de contrôles sécuritaires (contrôles routiers par exemple) ou d'enquêtes judiciaires.

### ÉTAT DE LA TECHNIQUE ANTÉRIEURE

D'après l'Organisation Mondiale de la Santé, le trafic de stupéfiants, est, en dollars, le troisième commerce en importance dans le monde, derrière ceux du pétrole et de l'alimentation mais juste avant ceux des armes et des médicaments.

Outre d'être à l'origine de réels problèmes sanitaires (décès par surdose, transmission d'agents infectieux du type VIH ou VHC) et sociaux, le trafic de stupéfiants génère une criminalité spécifique et la création de réseaux de type mafieux, avec leur contingent de guerres des gangs, de règlements de compte, etc.

La lutte contre le trafic de stupéfiants constitue donc un enjeu majeur.

Toutefois, elle implique que les services en charge de cette lutte et, en particulier, les services douaniers et policiers puissent disposer de moyens leur permettant de détecter sur sites la présence de stupéfiants, notamment dans l'air environnant.

A l'heure actuelle, la méthode la plus couramment utilisée pour détecter sur sites des vapeurs de stupéfiants est l'emploi de chiens renifleurs, dressés et entraînés à cet effet. Toutefois, l'utilisation de chiens renifleurs présente l'inconvénient de nécessiter une longue formation des chiens et de leurs maîtres et d'être inadaptée à des opérations prolongées en raison de ce que la durée d'attention des chiens est limitée. De plus, les chiens renifleurs ne sont pas capables de détecter les stupéfiants auxquels ils n'ont pas été sensibilisés.

On cherche donc de plus en plus à remplacer les chiens renifleurs par des appareils portables.

En matière d'appareils portables, il existe des appareils qui détectent les stupéfiants par spectrométrie à mobilité ionique. Ces appareils présentent l'avantage de permettre une détection rapide (en quelques dizaines de secondes au plus) et à faible coûts de la présence de stupéfiants. Par contre, autant la spectrométrie à mobilité ionique est une technique performante pour détecter la présence de stupéfiants sur des surfaces, autant elle se révèle être beaucoup moins fiable lorsqu'il s'agit de détecter des stupéfiants à l'état de vapeurs. En outre, cette technique nécessite la présence d'une source radioactive, ce qui implique que les appareils ne peuvent être utilisés que sous la responsabilité d'une personne compétente en radioprotection et par des personnes ayant des notions de radioprotection.

Depuis quelques années, le développement de capteurs capables de détecter en temps réel des espèces chimiques gazeuses est en plein essor. Le fonctionnement de ces capteurs est basé sur l'utilisation d'un film d'un matériau sensible, c'est-à-dire d'un matériau dont au moins une propriété physique P (masse, température, conductivité électrique, absorbance, fluorescence, etc) est modifiée au contact des molécules gazeuses recherchées, qui revêt un système apte à mesurer en temps réel toute variation de cette propriété physique et de mettre ainsi en évidence la présence des molécules gazeuses recherchées.

Les avantages des capteurs chimiques sont multiples : instantanéité des résultats, possibilité de miniaturisation et, donc, portabilité, maniabilité et autonomie importante, faibles coûts de fabrication et d'exploitation, etc. Par contre, il est évident que leurs performances sont extrêmement variables selon la nature du matériau sensible utilisé.

À ce jour, les matériaux sensibles qui ont été proposés pour la détection de stupéfiants sous forme de vapeurs se limitent à des polymères à empreinte moléculaire, contenant des complexes de lanthanides, et à des complexes antigènes/ anticorps faisant appel à des essais immunologiques (US 2003/0003587, **[1];** Stubbs et al., IEEE Sensors Journal 2005, 5, 335-339, **[2]** ; Frisk et al., Lab on a Chip 2008, 8, 1648-1657, **[3]**), ce qui est très peu.

Par ailleurs, un certain nombre d'auteurs s'est intéressé récemment aux propriétés de sorption des oligosilsesquioxanes polyédriques, aussi appelés silsesquioxanes oligomères polyédriques ou POSS, et à la possibilité de les utiliser comme matériaux sensibles dans des capteurs chimiques.

Ainsi, il a été proposé par Massera et al. (Sensors and Actuators B 2008, 129, 487-490, **[4]**) d'utiliser un copolymère d'oligosilsesquioxane octaédrique fonctionnalisé par des groupes isobutyle et de n-butylméthacrylate comme matériau sensible dans un capteur résistif pour détecter de l'ammoniac, des composés organiques volatils (COV) et de la vapeur d'eau.

Il a été proposé par Sirbuly et al. (Advanced Materials 2008, 20, 4724-4727, **[5]**) d'utiliser un matériau nanocomposite poreux constitué d'un oligosilsesquioxane octaédrique fonctionnalisé par des groupes propylammonium et de palladium comme matériau sensible dans un capteur optique pour détecter la présence d'hydrogène dans l'atmosphère. Le capteur ne s'est révélé être efficace que pour une teneur atmosphérique en hydrogène ne dépassant pas 10%, le signal émis par le capteur étant, en effet, saturé au-delà de cette teneur.

Il a encore été proposé par Hartmann et al. (US 7,998,415, **[6]**) d'utiliser des matériaux constitués d'une matrice polymère du type matrice d'un polycarbosilane, dans laquelle est dispersé, en tant que charge particulaire solide, un oligosilsesquioxane octaédrique fonctionnalisé par des groupes basiques à liaisons hydrogènes, comme matériaux sensibles dans des capteurs à ondes acoustiques de surface (ou capteurs SAW) pour détecter des vapeurs d'explosifs, telles que des vapeurs de dinitrotoluène, et des gaz innervants.

Par contre, à la connaissance des Inventeurs, l'utilisation d'oligosilsesquioxanes octaédriques pour détecter des stupéfiants et, en particulier, comme matériaux sensibles dans des capteurs chimiques destinés à détecter la présence de stupéfiants dans un milieu gazeux n'a jamais été proposée à ce jour.

Or, dans le cadre de leurs travaux sur le développement de capteurs chimiques destinés à la détection de stupéfiants, les Inventeurs ont constaté que les oligosilsesquioxanes octaédriques réagissent avec une très grande sensibilité aux vapeurs d'un certain nombre de stupéfiants dans un milieu gazeux et sont donc susceptibles de constituer des matériaux sensibles de choix pour des capteurs chimiques destinés à détecter la présence de ces stupéfiants dans un milieu gazeux.

Et c'est sur cette constatation qu'est basée la présente invention.

### EXPOSÉ DE L'INVENTION

L'invention a donc pour objet l'utilisation d'au moins un oligosilsesquioxane octaédrique (plus simplement appelé « POSS » dans ce qui suit) qui répond à la formule générale (I) ci-après : 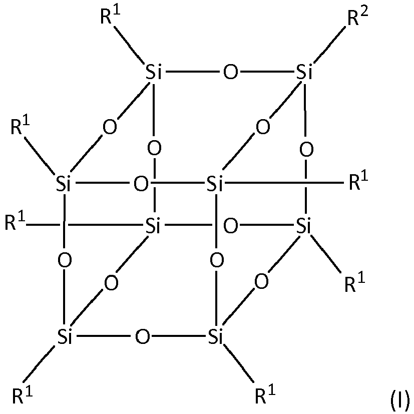 dans laquelle :
- soit R¹ et R² sont identiques entre eux et représentent un groupe de formule : 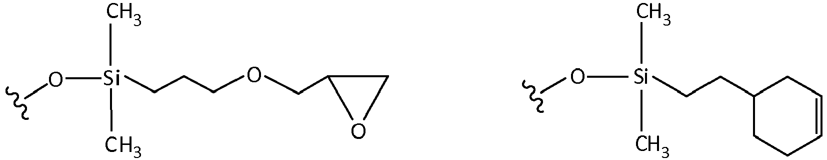 ou 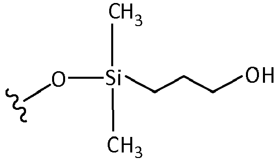
- soit R¹ et R² sont différents l'un de l'autre, auquel cas R¹ représente un groupe de formule : 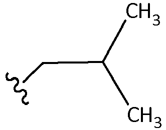 tandis que R² représente un groupe de formule : 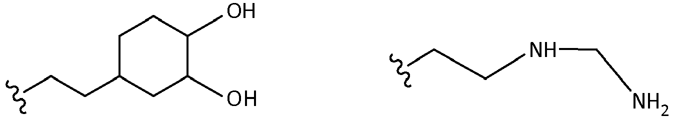 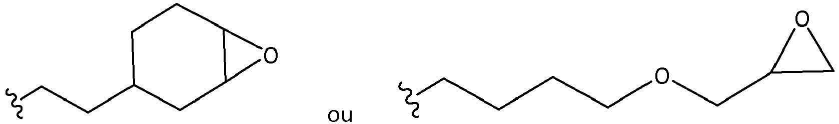
comme matériau sensible dans un capteur chimique pour détecter la présence dans un milieu gazeux d'au moins un composé choisi parmi les opiacés, la cocaïne, les cannabinoïdes, les amphétamines, le diéthylamide de l'acide lysergique, la cathine, la cathinone, les dérivés synthétiques de la cathinone, l'acide γ-hydroxybutyrique, la kétamine, le nitrite d'isoamyle, le nitrite de n-amyle, leurs isomères, leurs sels, leurs esters, leurs éthers, leurs précurseurs et leurs produits de dégradation.

Dans ce qui précède et ce qui suit, on entend par « *stupéfiant* », tout psychotrope dont la production, le commerce, le transport, l'importation, l'exportation, la détention, l'emploi et la consommation sont illicites ou sujets à une réglementation.

On entend par « *psychotrope* », toute substance chimique, d'origine naturelle ou synthétique, qui agit sur le système nerveux central en induisant des modifications de la perception, des sensations, de l'humeur ou de la conscience.

On entend par « *opiacé* », tout composé qui agit sur les récepteurs opiacés chez l'homme et qui est, soit naturellement présent dans l'opium comme : la morphine, la codéine ou la thébaïne, soit obtenu par synthèse comme l'héroïne, l'hydromorphone, l'hydrocodone, l'oxymorphone, l'oxycodone, la méthadone, la tilidine, le tramadol, la buprénorphine, le fentanyl ou la dihydrocodéine.

On entend par « *cannabinoïde* », tout composé qui agit sur les récepteurs cannabinoïdes chez l'homme et qui est, soit naturellement présent dans le cannabis comme : le Δ9-tétrahydrocannabinol (ou THC) ou la tétrahydrocannabivarine, soit obtenu par synthèse comme le 1-pentyl-3-(1-naphthoyl)indole (ou JWH-018), le 2-[(lR,3S)-3-hydroxycyclohexyl]-5-(2-méthyloctan-2-yl)phénol (ou CP 47,497), le 2-[(1*R*,3*S*)-3-hydroxycyclohexyl]-5-(1,1-diméthylhexyl)phénol (ou (C6)-CP 47,497), le 2-[(1*R*,3*S*)-3-hydroxycyclohexyl]-5-(2-méthyl-nonan-2-yl)phénol (ou (C8)-CP 47,497), le 2-[(1*R*,3*S*)-3-hydroxycyclohexyl]-5-(2-methyldecan-2-yl)phénol (ou (C9)-CP 47,497), ou le (6a*R*,10a*R*)-9-(hydroxyméthyl)-6,6-diméthyl-3-(2-méthyl-octan-2-yl)-6a,7,10,10a-tétrahydrobenzo[c]-chromèn-1-ol (ou HU-210).

On entend par « *amphétamine* », tout composé dérivé de la phényléthylamine par méthylation de l'atome de carbone situé en α du groupe amine de la phényléthylamine comme : l'amphétamine *sensu stricto,* la 4-fluoroamphétamine, la 4-méthylamphétamine (ou 4-MA), la méthamphétamine, la 3,4-méthylène-dioxy-*N-*méthylamphétamine (ou MDMA, plus connue sous le nom d'ecstasy), la 3,4-méthylènedioxy-*N*-éthylamphétamine (ou MDEA), la 3,4-méthylènedioxyamphétamine (ou MDA), ou la 2,5-diméthoxy-4-méthylamphétamine (ou DOM).

On entend par « *dérivé synthétique de la cathinone* », tout composé dérivé de la cathinone par substitution d'un ou plusieurs atomes de carbone du groupe phényle de la cathinone et/ou par substitution de l'atome d'azote du groupe amine de la cathinone comme : l'éphédrone (ou méthcathinone), la méthédrone (ou 4-méthoxy-méthcathinone), l'éthylcathinone, la butylone, la méphédrone, la méthylone, l'amphépramone, la métamfépramone, l'éthycathinone (ou éthylcathinone), ou la fléphédrone.

Par ailleurs, dans les groupes représentés ci-avant, le symbole « 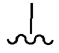 » matérialise la liaison covalente par laquelle ces groupes sont fixés sur le coeur octaédrique du POSS.

Le POSS susceptible d'être utilisé conformément à l'invention peut donc être l'un quelconque des POSS 1 à 7, qui répondent à la formule générale (I) ci-avant dans laquelle R¹ et R² sont tels que définis dans le tableau I ci-après.

**Tableau I**

| **POSS** | **R¹** | **R²** |
|---|---|---|
| **POSS 1** | 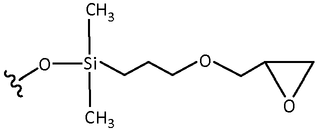 | 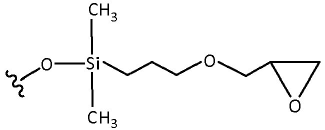 |
| **POSS 2** | 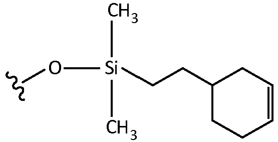 | 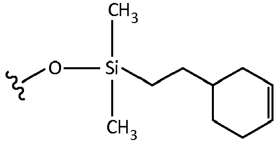 |
| **POSS 3** | 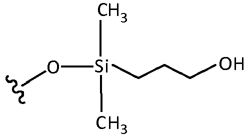 | 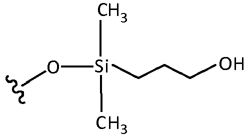 |
| **POSS 4** | 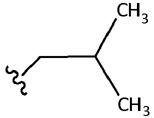 | 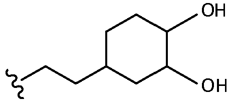 |
| **POSS 5** | 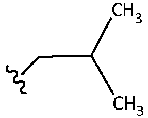 | 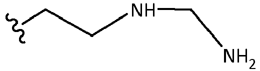 |
| **POSS 6** | 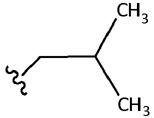 | 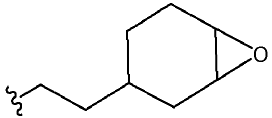 |
| **POSS 7** | 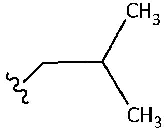 | 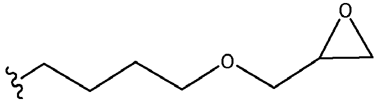 |

Ces POSS sont disponibles notamment auprès de la société Sigma-Aldrich sous les références : 593869 pour le POSS 1, 593974 pour le POSS 2, 594180 pour le POSS 3, 560286 pour le POSS 4, 560294 pour le POSS 5, 560316 pour le POSS 6 et 560324 pour le POSS 7.

Parmi ces POSS, préférence est donnée aux POSS 1, 3, 4 et 7.

Conformément à l'invention, le POSS tel que défini ci-avant est présent dans le capteur de préférence sous la forme d'un film mince qui recouvre l'une ou les deux faces d'un substrat convenablement choisi en fonction de la propriété physique dont les variations sont destinées à être mesurées par ce capteur.

En variante, ce POSS peut également être présent dans le capteur sous la forme d'un objet massif comme, par exemple, un cylindre présentant une certaine porosité de sorte à rendre accessible aux composés que l'on souhaite détecter l'ensemble des molécules de cet oligosilsesquioxane.

Lorsque le POSS tel que défini ci-avant se présente sous la forme d'un film mince, ce film présente, de préférence, une épaisseur de 1 nanomètre à 10 micromètres.

Un tel film peut être obtenu par l'une quelconque des techniques proposées à ce jour pour réaliser un film mince sur la surface d'un substrat et, notamment, par dépôt par pulvérisation, par dépôt à la tournette (ou « *spin coating* » en anglais), par dépôt à la goutte, par dépôt-évaporation (ou « *drop coating* » en anglais), par trempage-retrait (ou « *dip coating* » en anglais) ou encore par la technique de Langmuir-Blodgett, en utilisant une solution comprenant le POSS dans un solvant organique volatil tel que du chloroforme.

Le substrat ainsi que le système de mesure du capteur sont choisis en fonction de la propriété physique du POSS dont les variations induites par la présence des composés à détecter sont destinées à être mesurées par le capteur.

En l'espèce, les variations de masse des POSS tels que définis ci-avant, après dépôt sur un substrat piézoélectrique de type microbalance à quartz ou de type à ondes acoustiques de surface (ou SAW de « *Surface Acoustic Wave* »), se sont révélées particulièrement intéressantes à mesurer.

Aussi, le capteur est-il, de préférence, un capteur gravimétrique et, plus spécifiquement, un capteur à microbalance à quartz ou un capteur à ondes acoustiques de surface. Le principe de fonctionnement de ces capteurs a notamment été décrit par Sanchez-Pedrono et al. dans Analytica Chimica Acta, 1986, 182, 285, **[7],** pour les capteurs à microbalance à quartz et par Hoummady et al. dans Smart Materials and Structures 1997, 6, 647-657, **[8],** pour les capteurs SAW.

Par ailleurs, il est possible de réunir au sein d'un même dispositif ou *« multicapteur* », plusieurs capteurs élémentaires comprenant des matériaux sensibles différents les uns des autres, ou munis de substrats et de systèmes de mesure différents les uns des autres, l'essentiel étant que l'un au moins de ces capteurs comprenne un POSS tel que défini ci-avant.

L'utilisation des POSS tels que définis ci-avant en tant que matériaux sensibles dans des capteurs chimiques pour détecter des stupéfiants comme, par exemple, la cocaïne et le nitrite d'isoamyle, leurs isomères, leurs sels, leurs esters, leurs éthers, leurs précurseurs et leurs produits de dégradation comme, par exemple, le benzoate de méthyle qui est le produit de dégradation de la cocaïne en présence de vapeur d'eau, s'est révélée présenter de nombreux avantages.

En effet, outre que ces POSS présentent une très grande sensibilité vis-à-vis de l'ensemble de ces substances lorsqu'elles sont présentes dans un milieu gazeux, ils réagissent à la présence de ces substances de manière immédiate ou quasi immédiate, reproductible et réversible.

De par leur solubilité dans les solvants, ils peuvent de plus être aisément mis en oeuvre sous la forme de films minces, nécessitant une très faible quantité de POSS, et permettent de ce fait d'envisager la fabrication de capteurs miniaturisés, aisément transportables et manipulables sur tout type de sites, à des coûts compatibles avec une production de capteurs à une échelle industrielle.

À titre d'exemple qui ne fait pas partie de l'invention, un procédé permet de détecter la présence dans un milieu gazeux d'au moins un composé choisi parmi les opiacés, la cocaïne, les cannabinoïdes, les amphétamines, le diéthylamide de l'acide lysergique, la cathine, la cathinone, les dérivés synthétiques de la cathinone, l'acide γ-hydroxybutyrique, la kétamine, le nitrite d'isoamyle, le nitrite de n-amyle, leurs isomères, leurs sels, leurs esters, leurs éthers, leurs précurseurs et leurs produits de dégradation, qui comprend :
- une mise en contact du milieu gazeux avec un capteur chimique comprenant un matériau sensible ayant au moins une propriété physique qui est modifiée au contact du composé, dans laquelle le matériau sensible comprend au moins un POSS tel que défini ci-avant, le capteur fournissant une première réponse lorsque le composé n'est pas présent dans le milieu gazeux et fournissant une deuxième réponse lorsque le composé est présent dans le milieu gazeux, la deuxième réponse étant différente de la première réponse et correspondant à une modification de la propriété physique du matériau sensible au contact du composé ; et
- une mesure d'un changement de réponse du capteur et une corrélation du changement de réponse du capteur à la présence du composé dans le milieu gazeux.

Dans ce procédé, les caractéristiques particulières du POSS et du capteur chimique sont les mêmes que celles précédemment décrites l'utilisation de ce POSS.

D'autres caractéristiques et avantages de l'invention apparaîtront mieux à la lecture du complément de description qui suit, qui se rapporte à des exemples d'utilisation de POSS tels que définis ci-avant, comme matériaux sensibles dans des capteurs gravimétriques, et de démonstration des propriétés de ces capteurs.

### BRÈVE DESCRIPTION DES FIGURES

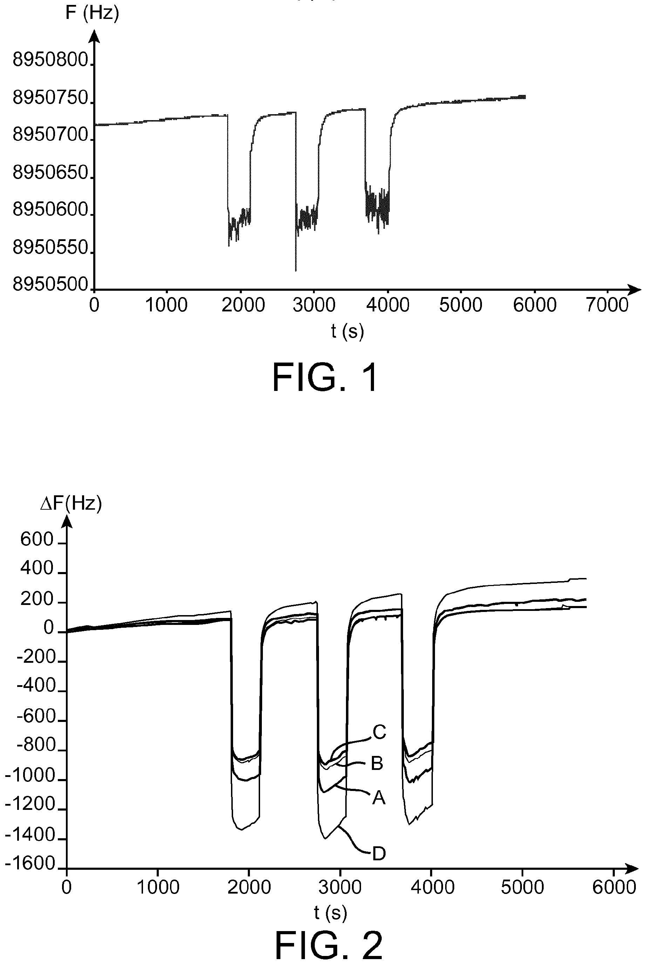

La figure 1 représente l'évolution de la fréquence de résonance (F), exprimée en hertz (Hz), en fonction du temps (t), exprimé en secondes, d'un premier capteur à microbalance à quartz comprenant un film mince de POSS 1, lorsque ce capteur est exposé successivement à l'air ambiant et à des vapeurs de benzoate de méthyle qui est un produit de dégradation de la cocaïne en présence de vapeur d'eau.
La figure 2 représente les variations de la fréquence de résonance (ΔF), exprimée en Hz, en fonction du temps (t), exprimé en secondes, de quatre autres capteurs à microbalance à quartz comprenant un film mince de POSS 4, lorsque ces capteurs sont exposés successivement à l'air ambiant et à des vapeurs de benzoate de méthyle.

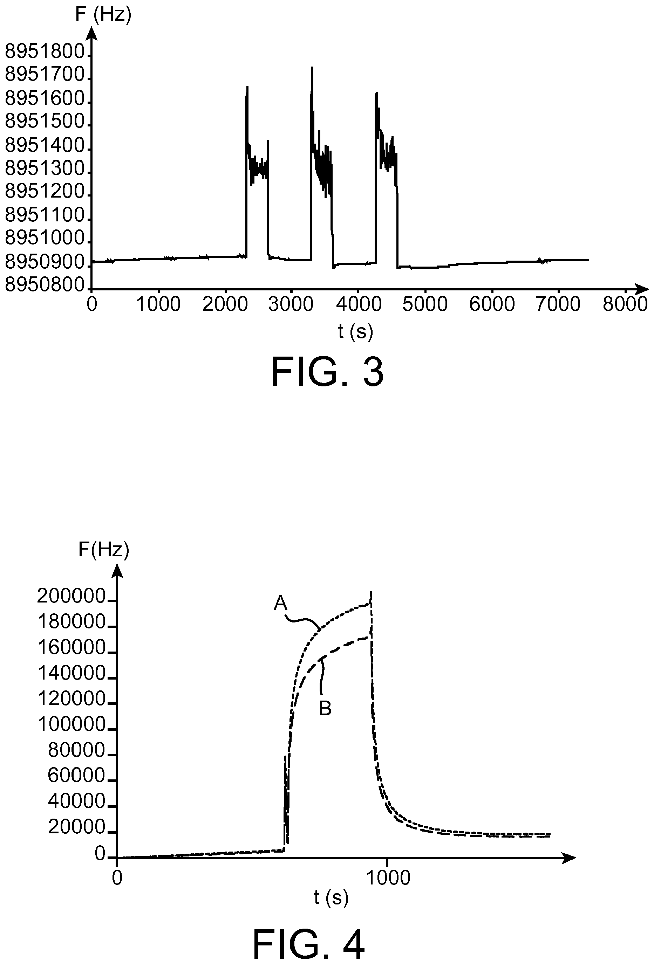

La figure 3 représente l'évolution de la fréquence de résonance (F), exprimée en Hz, en fonction du temps (t), exprimé en secondes, d'un autre capteur à microbalance à quartz, comprenant un film mince de POSS 1, lorsque ce capteur est exposé successivement à l'air ambiant et à des vapeurs de nitrite d'isoamyle qui est un des principaux constituants des poppers.
La figure 4 représente l'évolution de la fréquence de résonance (F), exprimée en Hz, en fonction du temps (t), exprimé en secondes, d'un premier capteur SAW comprenant un film mince de POSS 1, lorsque ce capteur est exposé successivement à l'air ambiant et à des vapeurs de benzoate de méthyle.

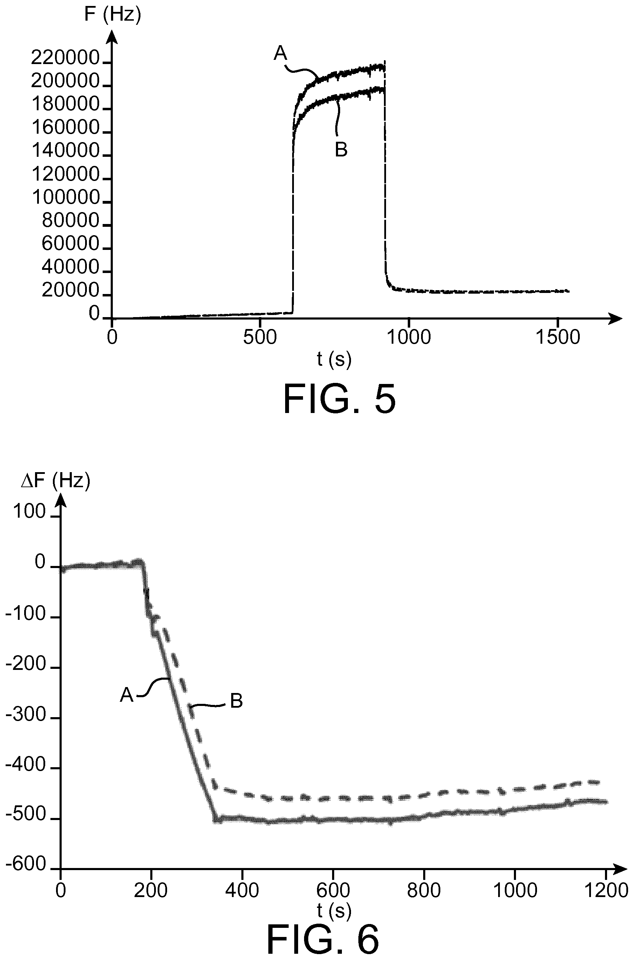

La figure 5 représente l'évolution de la fréquence de propagation de l'onde acoustique de surface (F), exprimée en Hz, en fonction du temps (t), exprimé en secondes, d'un autre capteur SAW, comprenant un film mince de POSS 1 lorsque ce capteur est exposé successivement à l'air ambiant et à des vapeurs de nitrite d'isoamyle.
La figure 6 représente les variations de la fréquence de résonance (ΔF), exprimée en Hz, en fonction du temps (t), exprimé en secondes, de deux autres capteurs à microbalance à quartz, comprenant un film mince de POSS 3 lorsque ces capteurs sont exposés successivement à l'air ambiant et à des vapeurs de cocaïne.

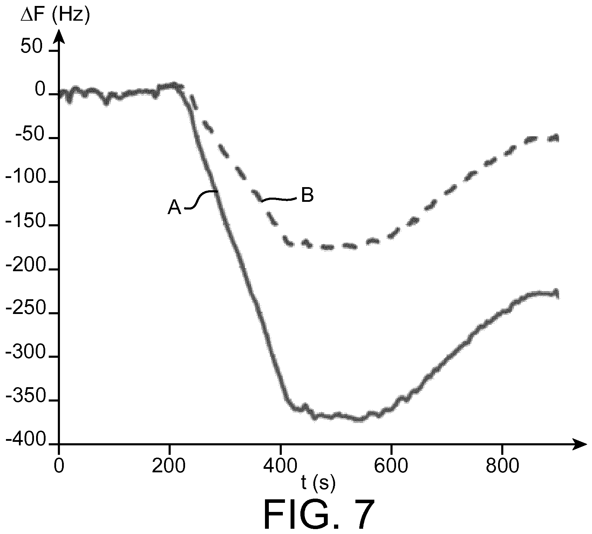

La figure 7 représente les variations de la fréquence de résonance (ΔF), exprimée en Hz, en fonction du temps (t), exprimé en secondes, de deux autres capteurs à microbalance à quartz, comprenant un film mince de POSS 7 lorsque ces capteurs sont exposés successivement à l'air ambiant et à des vapeurs de cocaïne.

### EXPOSÉ DÉTAILLÉ DE MODES DE RÉALISATION PARTICULIERS

### EXEMPLE 1: Détection de vapeurs de benzoate de méthyle (produit de dégradation de la cocaïne en présence de vapeur d'eau) par un premier capteur à microbalance à quartz

Dans cet exemple, on réalise un capteur à microbalance à quartz, dénommé « C1 » ci-après, en recouvrant les deux faces d'un quartz de coupe AT, de fréquence de résonance nominale de 9 MHz, muni de deux électrodes de mesure circulaires en or (modèle QA9RA-50, Ametek Precision Instruments), d'un film mince de POSS 1 (Sigma-Aldrich, référence 593869).

Le dépôt de ce film mince est réalisé en effectuant sur les deux faces du quartz 5 pulvérisations de 0,02 seconde chacune d'une solution de POSS 1 dans le chloroforme, de concentration égale à 10 g/L.

La variation de la fréquence de résonance du capteur C1 liée à ce dépôt est de 10 kHz.

Le capteur C1 est soumis à des tests de détection qui sont réalisés à 20°C et qui consistent à l'exposer successivement à :
- l'air ambiant pendant 30 minutes (soit 1 800 secondes) ;
- du benzoate de méthyle à une concentration de 350 ppm dans de l'air ambiant pendant 5 minutes (soit 300 secondes) ;
- l'air ambiant pendant 10 minutes (soit 600 secondes) ;
- du benzoate de méthyle à une concentration de 350 ppm dans de l'air ambiant pendant 5 minutes ;
- l'air ambiant pendant 10 minutes ;
- du benzoate de méthyle à une concentration de 350 ppm dans de l'air ambiant pendant 5 minutes ; et à
- l'air ambiant pendant 30 minutes.

La figure 1 illustre l'évolution de la fréquence de résonance (F) du capteur C1, exprimée en Hz, en fonction du temps (t), exprimé en secondes, telle qu'obtenue au cours de ces tests.

Comme le montre cette figure, on observe une chute de la fréquence de résonance du capteur C1 en présence de vapeurs de benzoate de méthyle, qui commence à se produire dès la mise en contact de ce capteur avec ces vapeurs. Cette chute de fréquence est réversible puisque le fait de remettre le capteur au contact de l'air ambiant entraîne immédiatement une remontée de sa fréquence de résonance.

Il convient de préciser que, dans le domaine des capteurs à microbalance à quartz, une variation de la fréquence de résonance d'un capteur est considérée comme significative et, donc, exploitable dès lors qu'elle est supérieure à trois fois le bruit de fond de ce capteur, soit environ 10 Hz dans le cas présent.

Or, comme le montre la figure 1, les chutes de la fréquence de résonance du capteur C1 induites par les expositions aux vapeurs de benzoate de méthyle sont très largement supérieures à cette valeur seuil puisqu'elles sont de 160 Hz pour les deux premières expositions et de 140 Hz pour la troisième exposition, ce qui signifie que le POSS présent dans le capteur est très sensible aux vapeurs de benzoate de méthyle.

### EXEMPLE 2 : Détection de vapeurs de benzoate de méthyle par quatre capteurs à microbalance à quartz

Dans cet exemple, on réalise 4 capteurs à microbalance à quartz, dénommés « C2 », « C3 », « C4 » et « C5 » ci-après, en recouvrant les deux faces de 4 quartz de coupe AT, de fréquence de résonance nominale de 100 MHz, muni de deux électrodes de mesure circulaires en or (modèle XA 3740 100 MHz, KVG Quartz Crystal Technology), d'un film mince de POSS 4 (Sigma-Aldrich, référence 560286).

Le dépôt de ce film mince est réalisé en effectuant sur les deux faces de chaque quartz 1 pulvérisation de 2 secondes d'une solution de POSS 4 dans le chloroforme, de concentration égale à 1,33 g/L.

La variation de la fréquence de résonance des capteurs C2 à C5 liée à ce dépôt est de : 186 kHz pour le capteur C2, 188 kHz pour le capteur C3, 177 kHz pour le capteur C4 et 264 kHz pour le capteur C5.

Les capteurs C2 à C5 sont soumis à des tests de détection qui sont réalisés à 20°C et qui consistent à les exposer successivement à :
- l'air ambiant pendant 30 minutes (soit 1 800 secondes) ;
- du benzoate de méthyle à une concentration de 350 ppm dans de l'air ambiant pendant 5 minutes (soit 300 secondes) ;
- l'air ambiant pendant 10 minutes (soit 600 secondes) ;
- du benzoate de méthyle à une concentration de 350 ppm dans de l'air ambiant pendant 5 minutes ;
- l'air ambiant pendant 10 minutes ;
- du benzoate de méthyle à une concentration de 350 ppm dans de l'air ambiant pendant 5 minutes ; et à
- l'air ambiant pendant 30 minutes.

La figure 2 illustre les variations de la fréquence de résonance (ΔF) des capteurs C2 à C5, exprimée en Hz, en fonction du temps (t), exprimé en secondes, telle qu'obtenue au cours de ces tests. Sur cette figure, la courbe A correspond au capteur C2 ; la courbe B correspond au capteur C3 ; la courbe C correspond au capteur C4 tandis que la courbe D correspond au capteur C5.

Comme le montre cette figure, on observe une chute de la fréquence de résonance des capteurs C2 à C5 en présence de vapeurs de benzoate de méthyle, qui commence à se produire dès la mise en contact de ces capteurs avec ces vapeurs. La diminution de la fréquence de résonance des capteurs est comprise entre 800 et 1400 Hz pour la première exposition, entre 900 et 1 600 Hz pour la deuxième exposition et entre 1 000 et 1400 Hz pour la troisième exposition, ce qui signifie que le POSS présent dans les capteurs C2 à C5 est très sensible aux vapeurs de benzoate de méthyle.

La désorption des vapeurs de benzoate de méthyle des couches minces de POSS est de surcroît très rapide puisque le fait de remettre les capteurs C2 à C5 au contact de l'air ambiant entraîne immédiatement une remontée de leur fréquence de résonance. La réversibilité de ces capteurs est donc excellente.

### EXEMPLE 3 : Détection de vapeurs de nitrite d'isoamyle (constituant des poppers) par un capteur à microbalance à quartz

Dans cet exemple, on utilise un capteur à microbalance à quartz, dénommé capteur « C6 » ci-après, identique à celui utilisé dans l'exemple 1 ci-avant.

Ce capteur est soumis à des tests de détection qui sont réalisés à 20°C et qui consistent à l'exposer successivement à :
- l'air ambiant pendant 30 minutes (soit 1 800 secondes) ;
- du nitrite d'isoamyle à une concentration de 35 000 ppm dans de l'air ambiant pendant 5 minutes (soit 300 secondes) ;
- l'air ambiant pendant 10 minutes (soit 600 secondes) ;
- du nitrite d'isoamyle à une concentration de 35 000 ppm dans de l'air ambiant pendant 5 minutes ;
- l'air ambiant pendant 10 minutes ;
- du nitrite d'isoamyle à une concentration de 35 000 ppm dans de l'air ambiant pendant 5 minutes ; et à
- l'air ambiant pendant 30 minutes.

La figure 3 illustre l'évolution de la fréquence de résonance (F) du capteur C6, exprimée en Hz, en fonction du temps (t), exprimé en secondes, telle qu'obtenue au cours de ces tests.

Comme le montre cette figure, on observe une augmentation de la fréquence de résonance du capteur C6 en présence de vapeurs de nitrite d'isoamyle, qui commence à se produire dès la mise en contact de ce capteur avec ces vapeurs et qui est réversible puisque le retour du capteur C6 à l'air ambiant se traduit immédiatement par un retour de sa fréquence de résonance à sa valeur initiale ou à une valeur proche de celle-ci.

Les augmentations de la fréquence de résonance du capteur C6 induites par les expositions aux vapeurs de nitrite d'isoamyle, qui sont respectivement de 450 Hz, 510 Hz et de 600 Hz, témoignent d'une très grande sensibilité du POSS présent dans ce capteur aux vapeurs de ce stupéfiant.

### EXEMPLE 4 : Détection de vapeurs de benzoate de méthyle par un capteur SAW

Dans cet exemple, on réalise deux capteurs SAW, dénommés « C7 » et « C8 » ci-après, de fréquence de résonance nominale de 433 MHz, en recouvrant les deux faces du substrat piézoélectrique de ce capteur d'un film mince de POSS 1 (Sigma-Aldrich, référence 593869).

Le dépôt de ce film mince est réalisé en effectuant 1 pulvérisation de 0,85 seconde d'une solution de POSS 1 dans le chloroforme, de concentration égale à 1,12 g/L.

La variation de la fréquence de résonance liée à ce dépôt est de 200 Hz.

Les capteurs C7 et C8 sont soumis à des tests de détection qui sont réalisés à 20°C et qui consistent à les exposer successivement à :
- l'air ambiant pendant 10 minutes (soit 600 secondes) ;
- du benzoate de méthyle à une concentration de 350 ppm dans de l'air ambiant pendant 5 minutes (soit 300 secondes) ; et à
- l'air ambiant pendant 10 minutes.

La figure 4 illustre l'évolution de la fréquence de résonance (F) des capteurs C7 et C8, exprimée en Hz, en fonction du temps (t), exprimé en secondes, telle qu'obtenue au cours de ces tests. Sur cette figure, la courbe A correspond au capteur C7 tandis que la courbe B correspond au capteur C8.

Comme le montre cette figure, on observe une augmentation de la fréquence de résonance des capteurs C7 et C8 en présence de vapeurs de benzoate de méthyle, qui commence à se produire dès la mise en contact de ces capteurs avec ces vapeurs et qui est réversible puisque le retour des capteurs à l'air ambiant se traduit immédiatement par un retour de leur fréquence de résonance à une valeur proche de sa valeur initiale.

L'augmentation de la fréquence de résonance des capteurs C7 et C8 induite par l'exposition de ces capteurs aux vapeurs de benzoate de méthyle est de l'ordre de 160 000 à 180 000 Hz et témoigne d'une très grande sensibilité du POSS présent dans ces capteurs aux vapeurs de ce composé.

### EXEMPLE 5 : Détection de vapeurs de nitrite d'isoamyle par un capteur SAW

Dans cet exemple, on utilise deux capteurs SAW, dénommés « C9 » et « C10 » ci-après, identiques aux capteurs C7 et C8 utilisés dans l'exemple 4 ci-avant.

Les capteurs C9 et C10 sont soumis à des tests de détection qui sont réalisés à 20°C et qui consistent à les exposer successivement à :
- l'air ambiant pendant 10 minutes (soit 600 secondes) ;
- du nitrite d'isoamyle à une concentration de 35 000 ppm dans de l'air ambiant pendant 5 minutes (soit 300 secondes) ; et à
- l'air ambiant pendant 10 minutes.

La figure 5 illustre l'évolution de la fréquence de résonance (F) des capteurs C9 et C10, exprimée en Hz, en fonction du temps (t), exprimé en secondes, telle qu'obtenue au cours de ces tests. Sur cette figure, la courbe A correspond au capteur C9 tandis que la courbe B correspond au capteur C10.

La figure 5 montre que l'exposition des capteurs C9 et C10 à des vapeurs de nitrite d'isoamyle se traduit par une augmentation de la fréquence de résonance de ces capteurs. Cette augmentation de fréquence de résonance est proche de 200 000 Hz et témoigne d'une très grande sensibilité du POSS présent dans les capteurs C9 et C10 aux vapeurs de nitrite d'isoamyle.

Là également, l'augmentation de la fréquence de résonance des capteurs C9 et C10 est réversible puisque le retour de ces capteurs à l'air ambiant se traduit immédiatement par un retour de leur fréquence de résonance à une valeur proche de sa valeur initiale.

### EXEMPLE 6 : Détection de vapeurs de cocaïne par deux capteurs à microbalance à quartz

Dans cet exemple, on réalise 2 capteurs à microbalance à quartz, dénommés « C11 » et « C12 » ci-après, en recouvrant les deux faces de 2 quartz de coupe AT, de fréquence de résonance nominale de 100 MHz, munis de deux électrodes de mesure circulaires en or (modèle XA 3740 100 MHz, KVG Quartz Crystal Technology), d'un film mince de POSS 3 (Sigma-Aldrich, référence 594180).

Le dépôt de ce film mince est réalisé en effectuant sur les deux faces de chaque quartz 1 pulvérisation de 0,6 seconde d'une solution de POSS 3 dans le chloroforme, de concentration égale à 1,23 g/L.

La variation de la fréquence de résonance des capteurs C11 et C12 liée à ce dépôt est de : 188 kHz pour le capteur C11, 199 kHz pour le capteur C12.

Les capteurs C11 et C12 sont soumis à des tests de détection qui sont réalisés à 20°C et qui consistent à les exposer successivement à :
- l'air ambiant pendant 3 minutes (soit 180 secondes) ;
- des vapeurs de cocaïne à la pression de vapeur dans de l'air ambiant pendant 2 minutes et 40 secondes (soit 160 secondes) ;
- l'air ambiant pendant 14 minutes et 20 secondes (soit 860 secondes) ;

La figure 6 illustre les variations de la fréquence de résonance (ΔF) des capteurs C11 et C12, exprimée en Hz, en fonction du temps (t), exprimé en secondes, telle qu'obtenue au cours de ces tests. Sur cette figure, la courbe A correspond au capteur C11 tandis que la courbe B correspond au capteur C12.

Comme le montre cette figure, on observe une chute de la fréquence de résonance des capteurs C11 et C12 en présence de vapeurs de cocaïne, qui commence à se produire dès la mise en contact de ces capteurs avec ces vapeurs. La diminution de la fréquence de résonance des capteurs est comprise entre 400 et 500 Hz, ce qui signifie que le POSS présent dans les capteurs C11 et C12 est très sensible aux vapeurs de cocaïne.

### EXEMPLE 7 : Détection de vapeurs de cocaïne par deux capteurs à microbalance à quartz

Dans cet exemple, on réalise 2 capteurs à microbalance à quartz, dénommés « C13 » et « C14 » ci-après, en recouvrant les deux faces de 2 quartz de coupe AT, de fréquence de résonance nominale de 100 MHz, munis de deux électrodes de mesure circulaires en or (modèle XA 3740 100 MHz, KVG Quartz Crystal Technology), d'un film mince de POSS 7 (Sigma-Aldrich, référence 590324).

Le dépôt de ce film mince est réalisé en effectuant sur les deux faces de chaque quartz 1 pulvérisation de 0,5 seconde d'une solution de POSS 7 dans le chloroforme, de concentration égale à 1,01 g/L.

La variation de la fréquence de résonance des capteurs C13 et C14 liée à ce dépôt est de : 188 kHz pour le capteur C13, 199 kHz pour le capteur C14.

Les capteurs C13 et C14 sont soumis à des tests de détection qui sont réalisés à 20°C et qui consistent à les exposer successivement à :
- l'air ambiant pendant 3 minutes 20 secondes (soit 200 secondes) ;
- des vapeurs de cocaïne à la pression de vapeur dans de l'air ambiant pendant 3 minutes et 20 secondes (soit 200 secondes) ;
- l'air ambiant pendant 6 minutes et 20 secondes (soit 500 secondes).

La figure 7 illustre les variations de la fréquence de résonance (ΔF) des capteurs C13 et C14, exprimée en Hz, en fonction du temps (t), exprimé en secondes, telle qu'obtenue au cours de ces tests. Sur cette figure, la courbe A correspond au capteur C13 tandis que la courbe B correspond au capteur C14.

Comme le montre cette figure, on observe une chute de la fréquence de résonance des capteurs C13 et C14 en présence de vapeurs de cocaïne, qui commence à se produire dès la mise en contact de ces capteurs avec ces vapeurs. La diminution de la fréquence de résonance des capteurs est comprise entre 400 et 500 Hz, ce qui signifie que le POSS présent dans les capteurs C13 et C14 est très sensible aux vapeurs de cocaïne.

### RÉFÉRENCES CITÉES

**[1]** US 2003/0003587
**[2]** Stubbs et al., IEEE Sensors Journal 2005, 5, 335-339
**[3]** Frisk et al., Lab on a Chip 2008, 8, 1648-1657
**[4]** Massera et al., Sensors and Actuators B 2008, 129, 487-490
**[5]** Sirbuly et al., Advanced Materials 2008, 20, 4724-4727
**[6]** US 7,998,415
**[7]** Sanchez-Pedrono et al., Analytica Chimica Acta, 1986, 1986, 285
**[8]** Hoummady et al., Smart Materials and Structures 1997, 6, 647-657

## Revendications

1. Utilisation d'au moins un oligosilsesquioxane octaédrique de formule générale (I) ci-après : 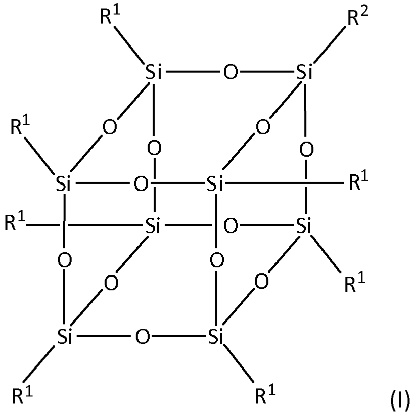 dans laquelle :
- soit R¹ et R² sont identiques entre eux et représentent un groupe de formule : 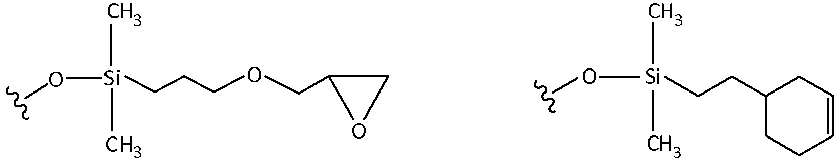 ou 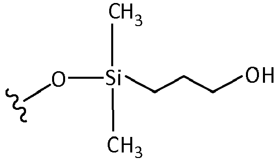
- soit R¹ et R² sont différents l'un de l'autre, auquel cas R¹ représente un groupe de formule : 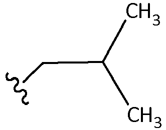 tandis que R² représente un groupe de formule : 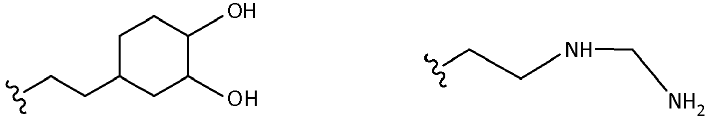 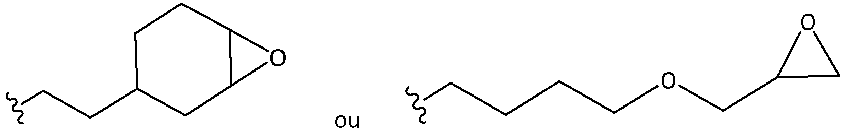 comme matériau sensible dans un capteur chimique pour détecter la présence dans un milieu gazeux d'au moins un composé choisi parmi les opiacés, la cocaïne, les cannabinoïdes, les amphétamines, le diéthylamide de l'acide lysergique, la cathine, la cathinone, les dérivés synthétiques de la cathinone, l'acide γ-hydroxybutyrique, la kétamine, le nitrite d'isoamyle, le nitrite de n-amyle, leurs isomères, leurs sels, leurs esters, leurs éthers, leurs précurseurs et leurs produits de dégradation.

2. Utilisation selon la revendication 1, dans laquelle l'oligosilsesquioxane octaédrique répond à la formule générale (I) dans laquelle :
- R¹ et R² représentent un groupe de formule ci-après : 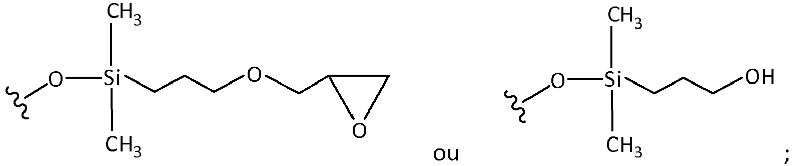 ou
- R¹ représente un groupe de formule ci-après : 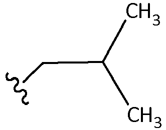 tandis que R² représente un groupe de formule ci-après :

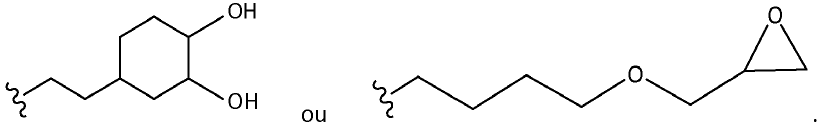

3. Utilisation selon la revendication 1 ou la revendication 2, dans laquelle l'oligosilsesquioxane octaédrique est utilisé sous la forme d'un film mince recouvrant l'une ou les deux faces d'un substrat du capteur.

4. Utilisation selon la revendication 3, dans laquelle le film mince mesure de 1 nanomètre à 10 micromètres d'épaisseur.

5. Utilisation selon l'une quelconque des revendications 1 à 4, dans laquelle le capteur chimique est un capteur gravimétrique.

6. Utilisation selon la revendication 5, dans laquelle le capteur chimique est un capteur à microbalance à quartz.

7. Utilisation selon l'une quelconque des revendications 1 à 5, dans laquelle le capteur chimique est un capteur à ondes acoustiques de surface.

8. Utilisation selon l'une quelconque des revendications 1 à 7, dans laquelle le composé est la cocaïne, le nitrite d'isoamyle ou le benzoate de méthyle.

## Patentansprüche

1. Verwendung wenigstens eines octaedrischen Oligosilsesquioxans mit der nachstehenden allgemeinen Formel (I): 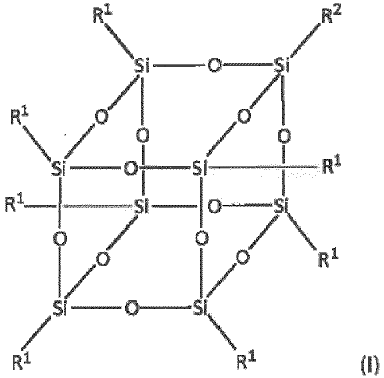 wobei:
- entweder R¹ und R² identisch zueinander sind und eine Gruppe mit folgender Formel darstellen: 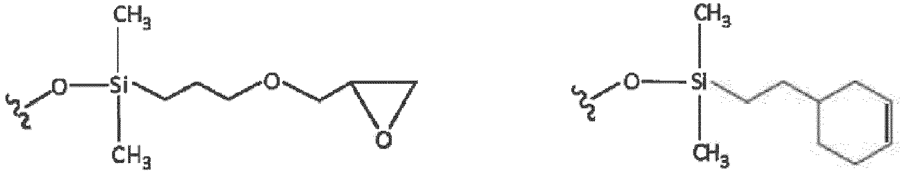 oder 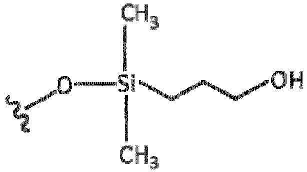
- oder R¹ und R² voneinander verschieden sind, in welchem Fall R¹ eine Gruppe mit folgender Formel darstellt: 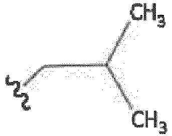 während R² eine Gruppe mit folgender Formel darstellt: 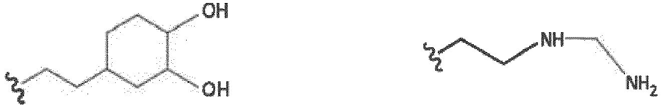 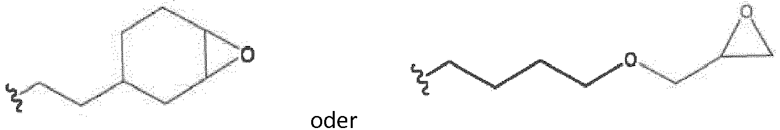 als empfindliches Material in einem chemischen Sensor zur Detektion des Vorliegens wenigstens einer Verbindung, welche gewählt ist aus Opiaten, Kokain, Cannabinoiden, Amphetaminen, Lysergsäure-Diethylamid, Cathin, Cathinon, synthetischen Derivaten von Cathinon, Y-Hydroxybuttersäure, Ketamin, Isoamylnitrit, *n*-Amylnitrit, deren Isomere, deren Salze, deren Ester, deren Ether, deren Vorläufer und deren Abbauprodukte in einem gasförmigen Medium.

2. Verwendung nach Anspruch 1, bei welcher das octaedrische Oligosilsesquioxan der allgemeinen Formel (I) entspricht, wobei:
- R¹ und R² eine Gruppe mit nachstehender Formel darstellen: 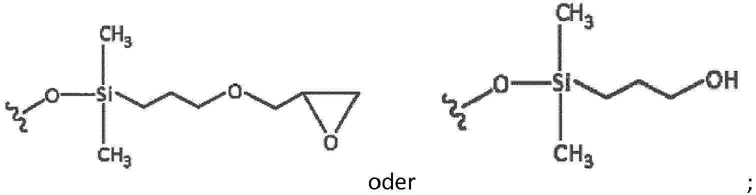 oder
- R¹ eine Gruppe mit nachstehender Formel darstellt: 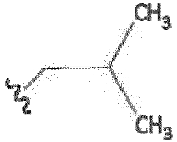 während R² eine Gruppe mit nachstehender Formel darstellt:

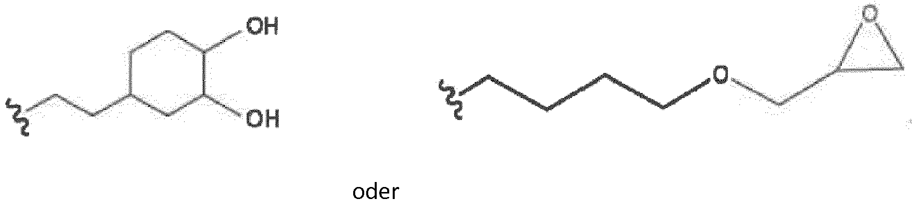

3. Verwendung nach Anspruch 1 oder Anspruch 2, bei welcher das octaedrische Oligosilsesquioxan in Form eines dünnen Films verwendet wird, der eine oder beide Seiten eines Substrats des Sensors bedeckt.

4. Verwendung nach Anspruch 3, bei welcher der dünne Film eine Dicke von 1 Nanometer bis 10 Mikrometer aufweist.

5. Verwendung nach einem der Ansprüche 1 bis 4, bei welcher der chemische Sensor ein gravimetrischer Sensor ist.

6. Verwendung nach Anspruch 5, bei welcher der chemische Sensor ein Quarzkristall-Mikrowaagen-Sensor ist.

7. Verwendung nach einem der Ansprüche 1 bis 5, bei welcher der chemische Sensor ein akustischer Oberflächenwellensensor ist.

8. Verwendung nach einem der Ansprüche 1 bis 7, bei welcher die Verbindung Kokain, Isoamylnitrit oder Methylbenzoat ist.

## Claims

1. Use of at least one octahedral oligosilsesquioxane of general formula (I) hereinafter: 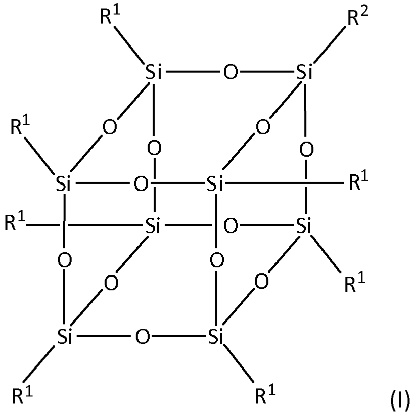 wherein:
- either R¹ and R² are identical to each other and represent a group of formula: 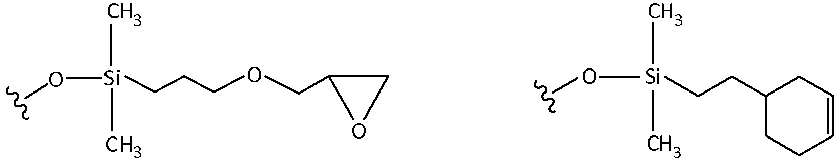 or 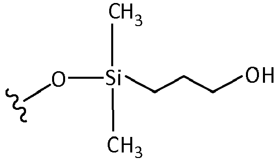
- or R¹ and R² are different from one another, in which case R¹ represents a group of formula: 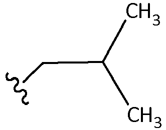 while R² represents a group of formula: 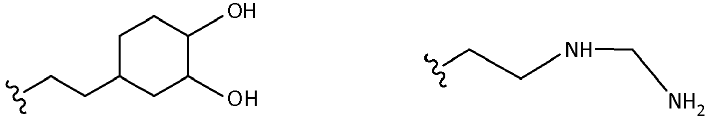 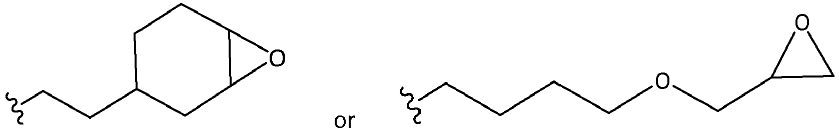 as sensitive material in a chemical sensor for detecting the presence, in a gas medium, of at least one compound selected from opiates, cocaine, cannabinoids, amphetamines, lysergic acid diethylamide, cathine, cathinone, the synthetic derivatives of cathinone, γ-hydroxybutyric acid, ketamine, isoamyl nitrite, n-amyl nitrite, the isomers thereof, the salts thereof, the esters thereof, the ethers thereof, the precursors thereof, and the breakdown products thereof.

2. Use according to claim 1, wherein the octahedral oligosilsesquioxane has general formula (I) wherein:
- R¹ and R² represent a group of formula hereinafter: 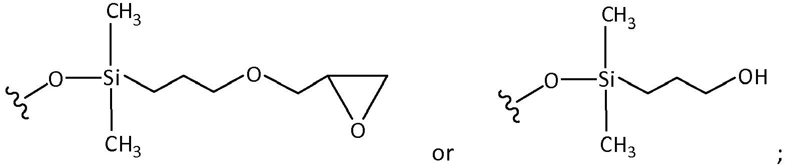 or
- R¹ represents a group of formula hereinafter: 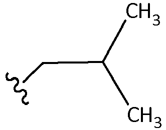 while R² represents a group of formula hereinafter:

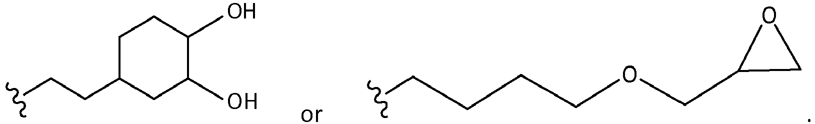

3. Use according to claim 1 or claim 2, wherein the octahedral oligosilsesquioxane is used in the form of a thin film covering one or both faces of a substrate of the sensor.

4. Use according to claim 3, wherein the thin film measures from 1 nanometre to 10 micrometres in thickness.

5. Use according to any of claims 1 to 4, wherein the chemical sensor is a gravimetric sensor.

6. Use according to claim 5, wherein the chemical sensor is a quartz crystal microbalance sensor.

7. Use according to any of claims 1 to 5, wherein the chemical sensor is a surface acoustic wave sensor.

8. Use according to any of claims 1 to 7, wherein the compound is cocaine, isoamyl nitrite or methyl benzoate.
